# EUROPEAN PATENT APPLICATION

(11) **EP 3 492 173 A2**
(43) Date of publication of application: **05.06.2019**
(21) Application number: 18213457.7
(22) Date of filing: 12.06.2007
(51) Int. Cl.: B02C 19/00, C12P 7/06, C12P 7/10

(54) **FIBROUS MATERIALS AND COMPOSITIONS**

(30) Priority: 15.06.2006 US 45395106
(62) Divisional of application: 16189685.7
(71) Applicant: Xyleco, Inc., Wakefield, MA 01880-6248 (US)
(72) Inventor: MEDOFF, Marshall, Brookline, MA 02146 (US)
(74) Representative: von Füner, Nicolai

(57) **Abstract**

The invention relates to a method comprising mechanically treating a lignocellulosic or cellulosic material and passing the mechanically treated material through a first screen having an opening size of 3.175 mm (1/8 inch) or less, to provide a screened fibrous material, and combining the screened fibrous material with a biodegradable polymer.

## Description

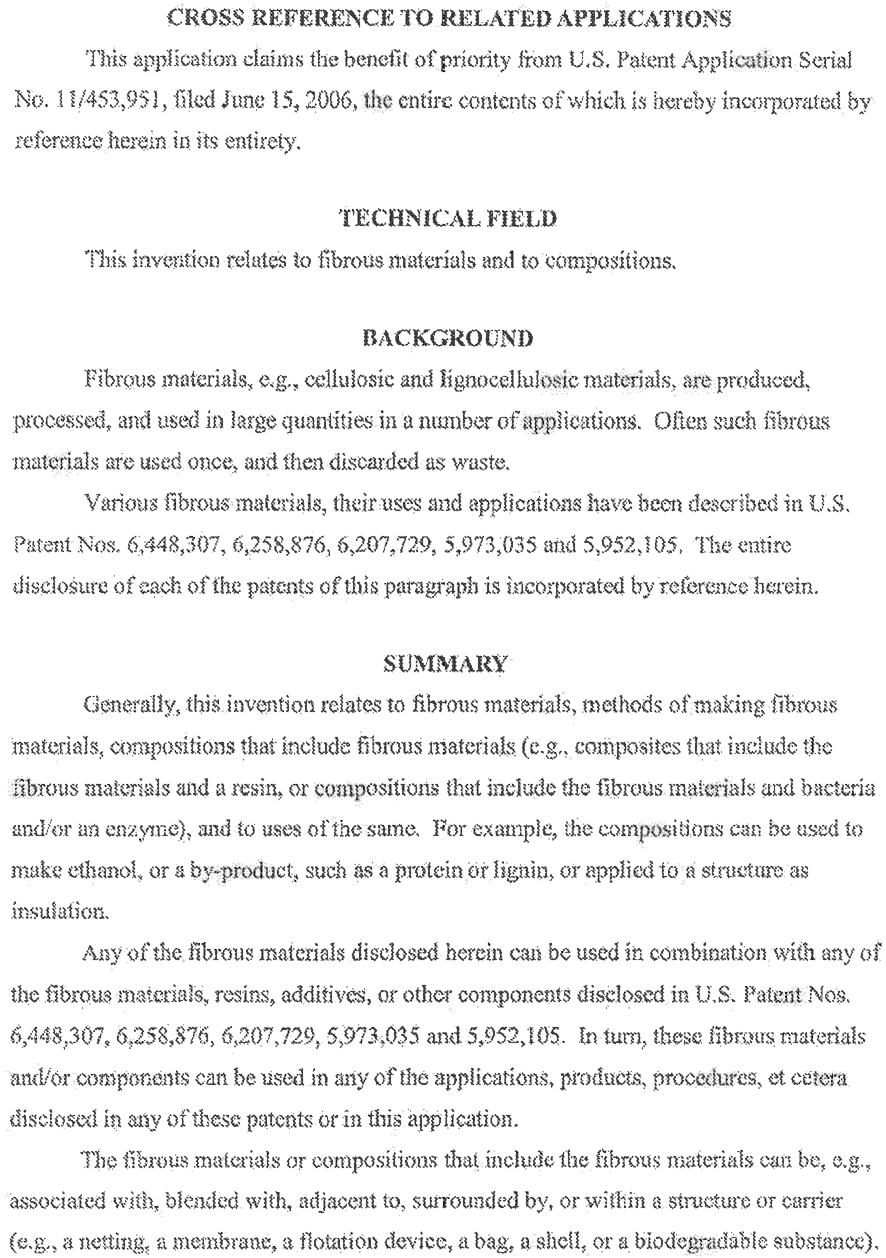

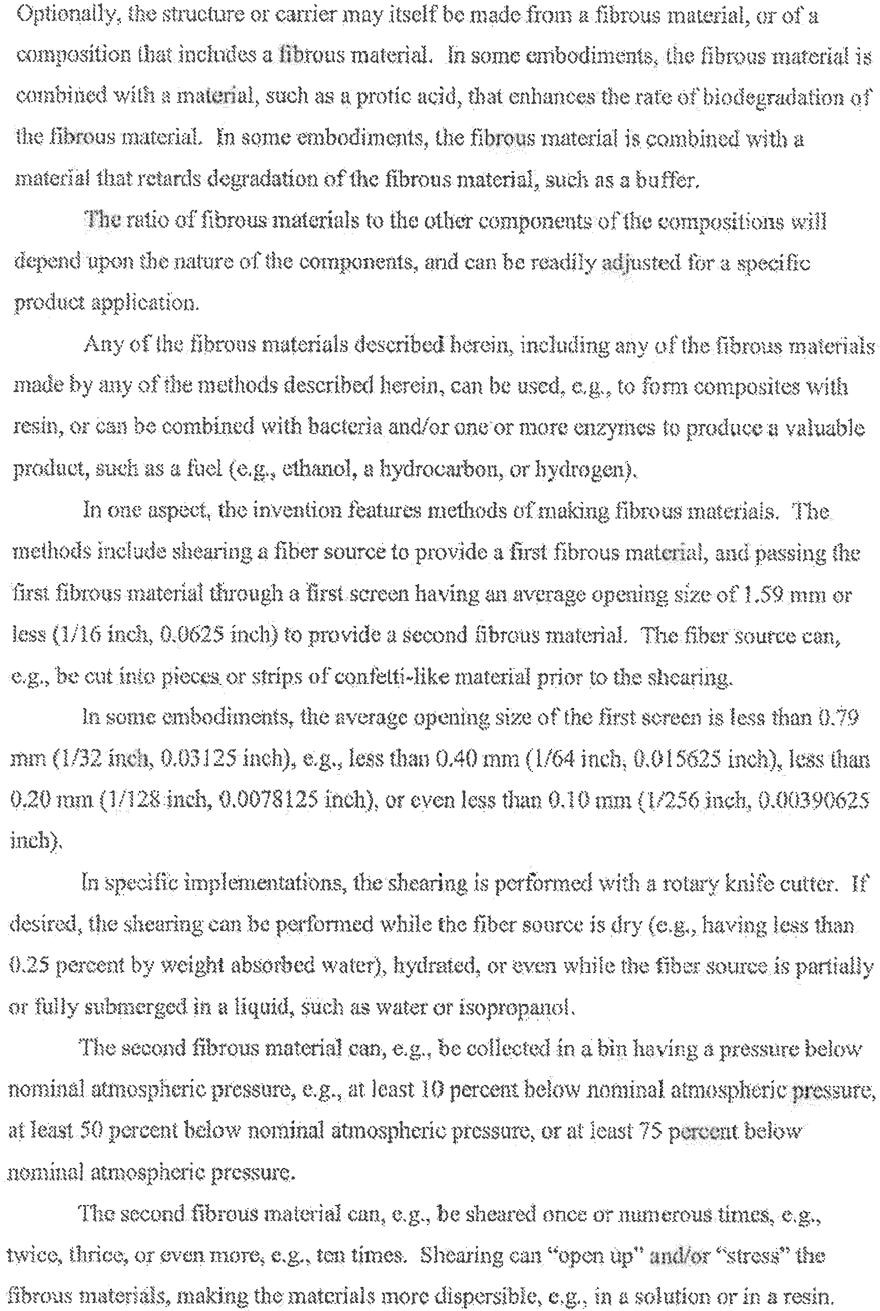

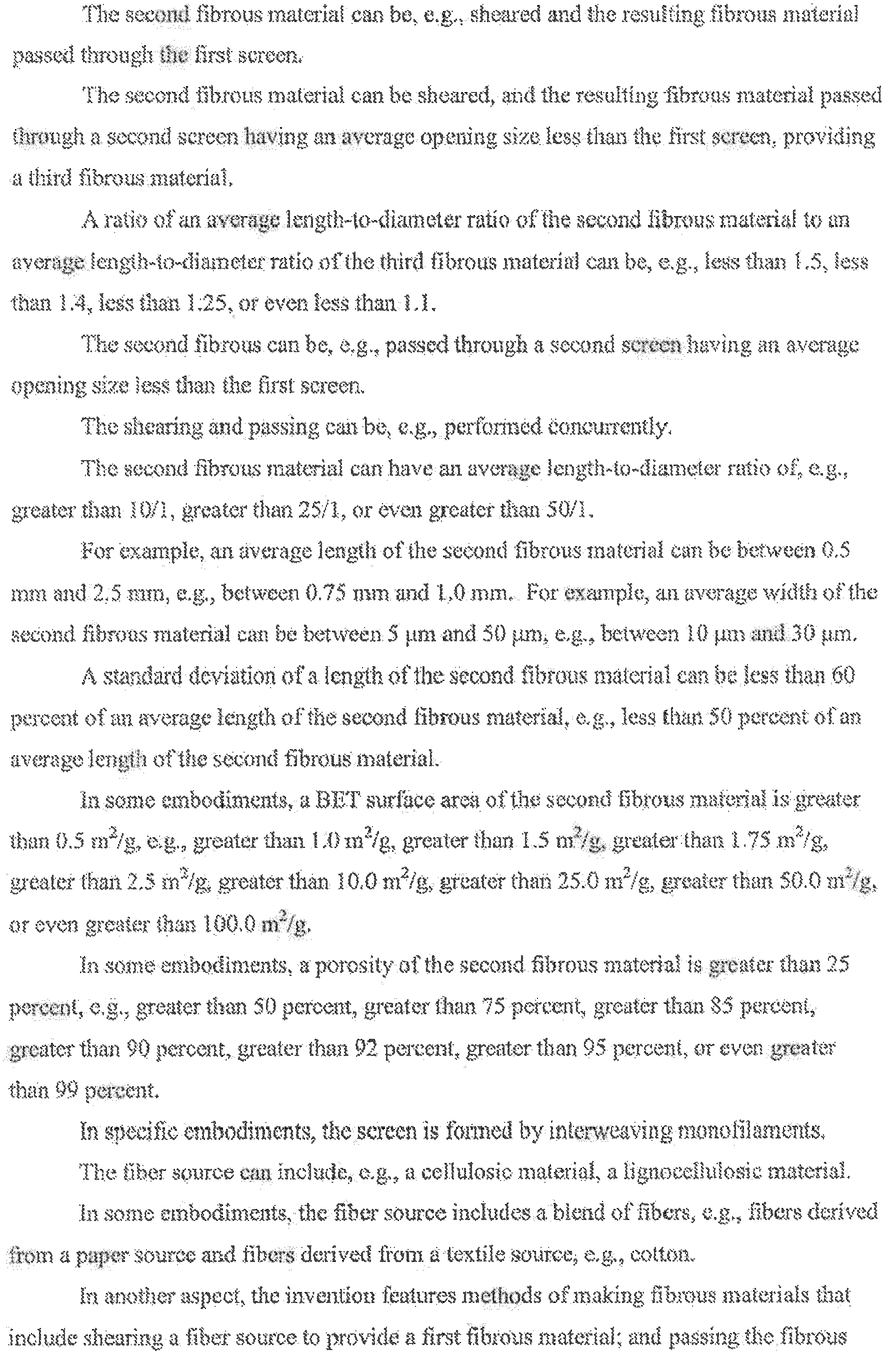

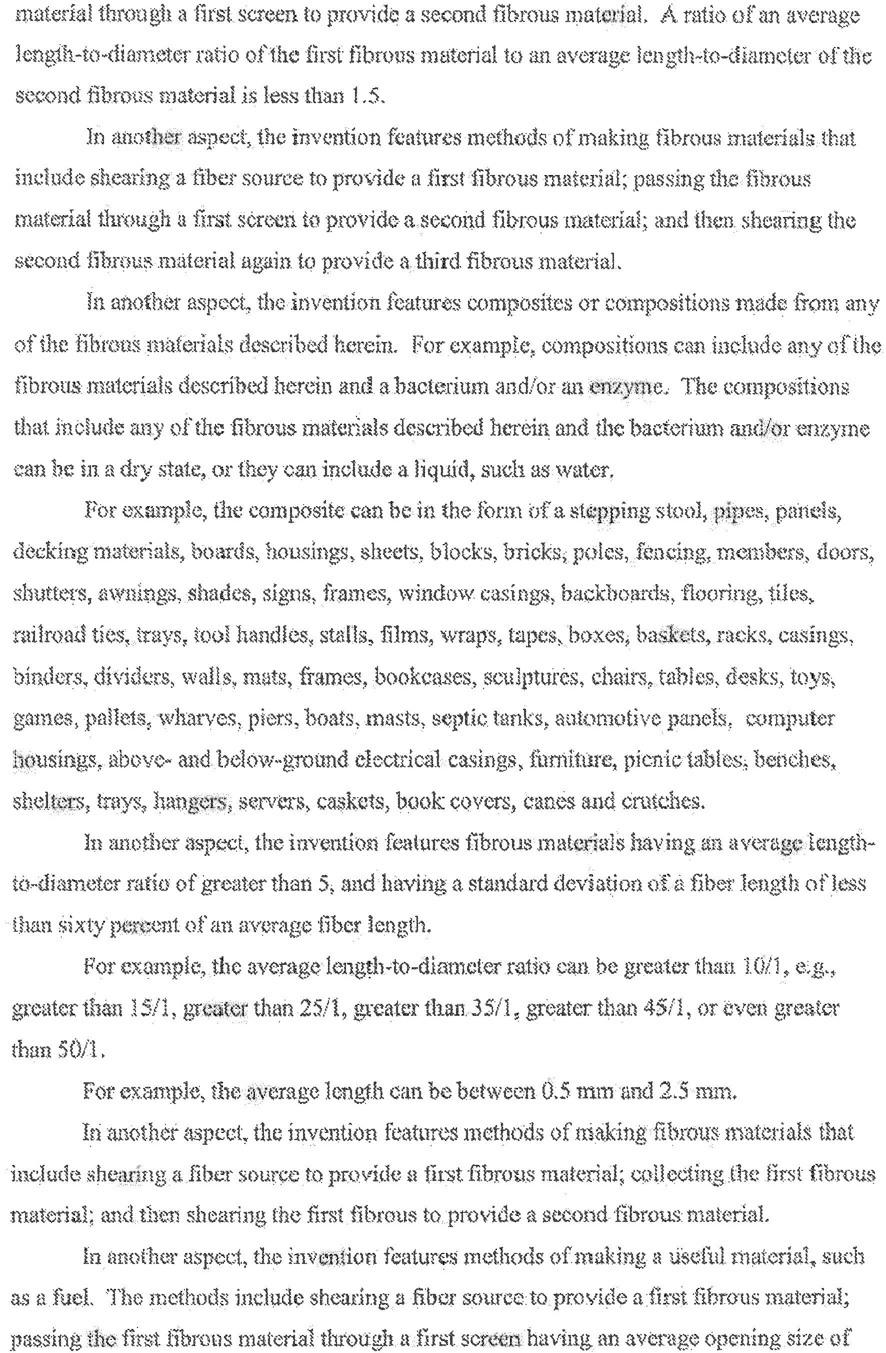

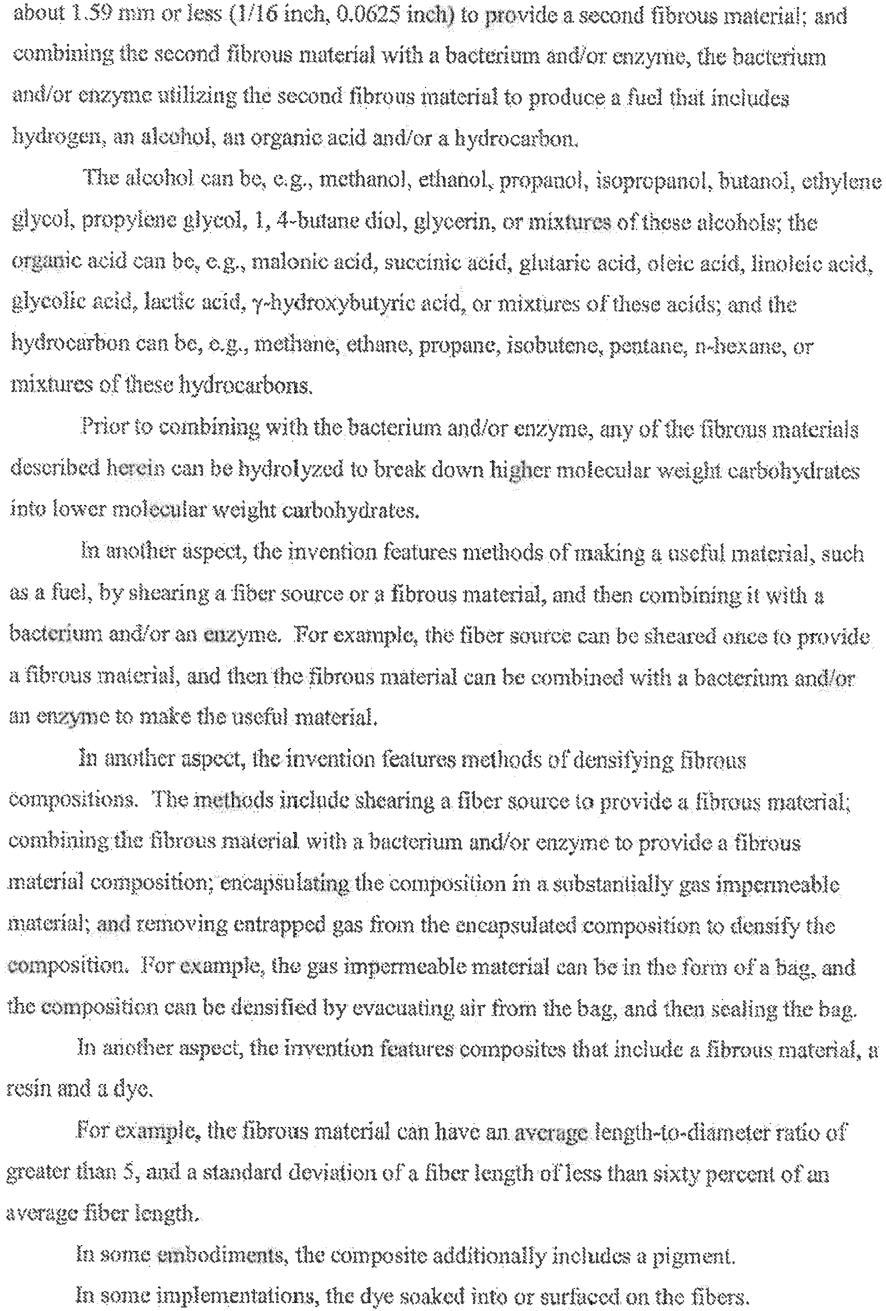

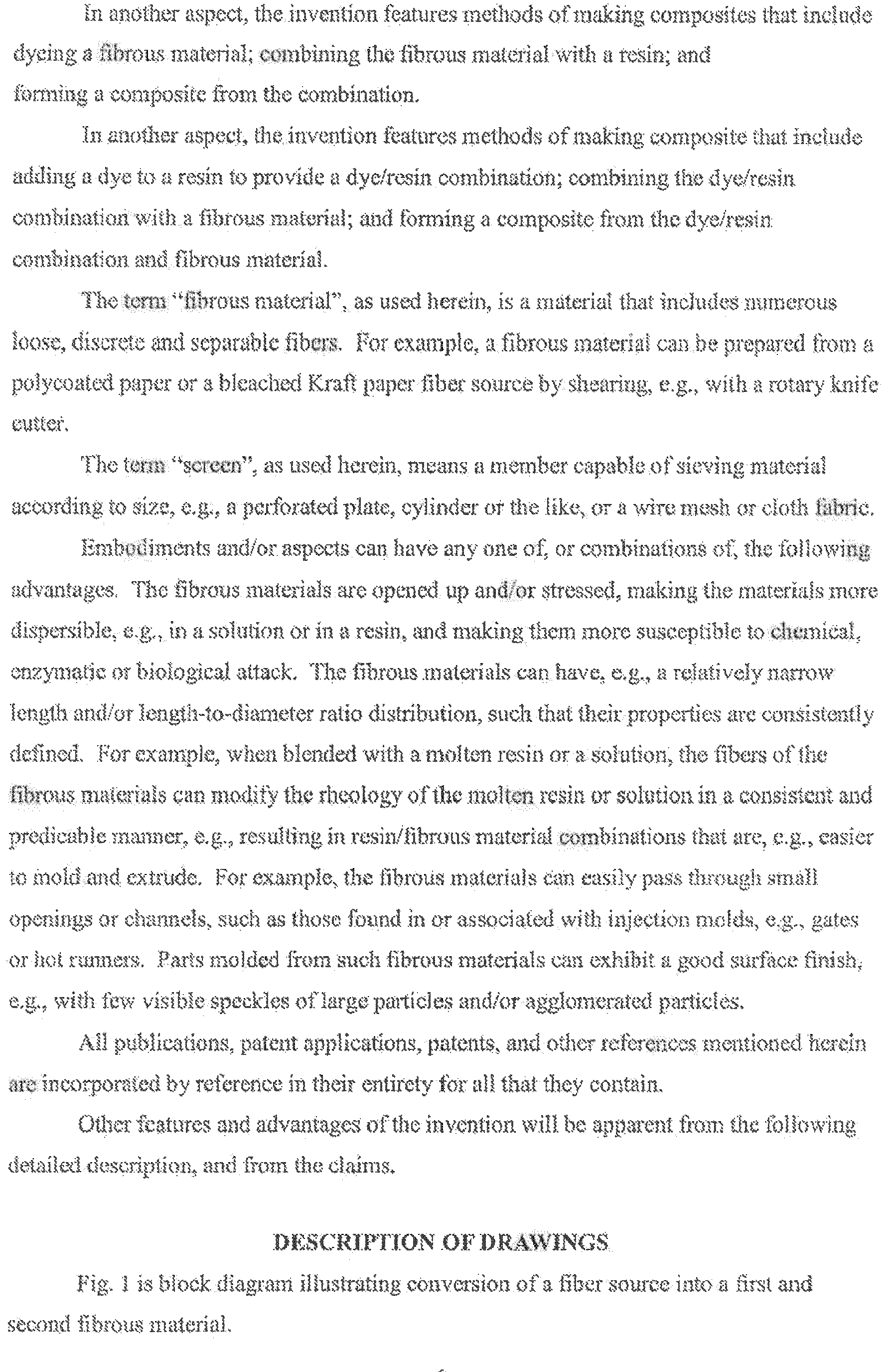

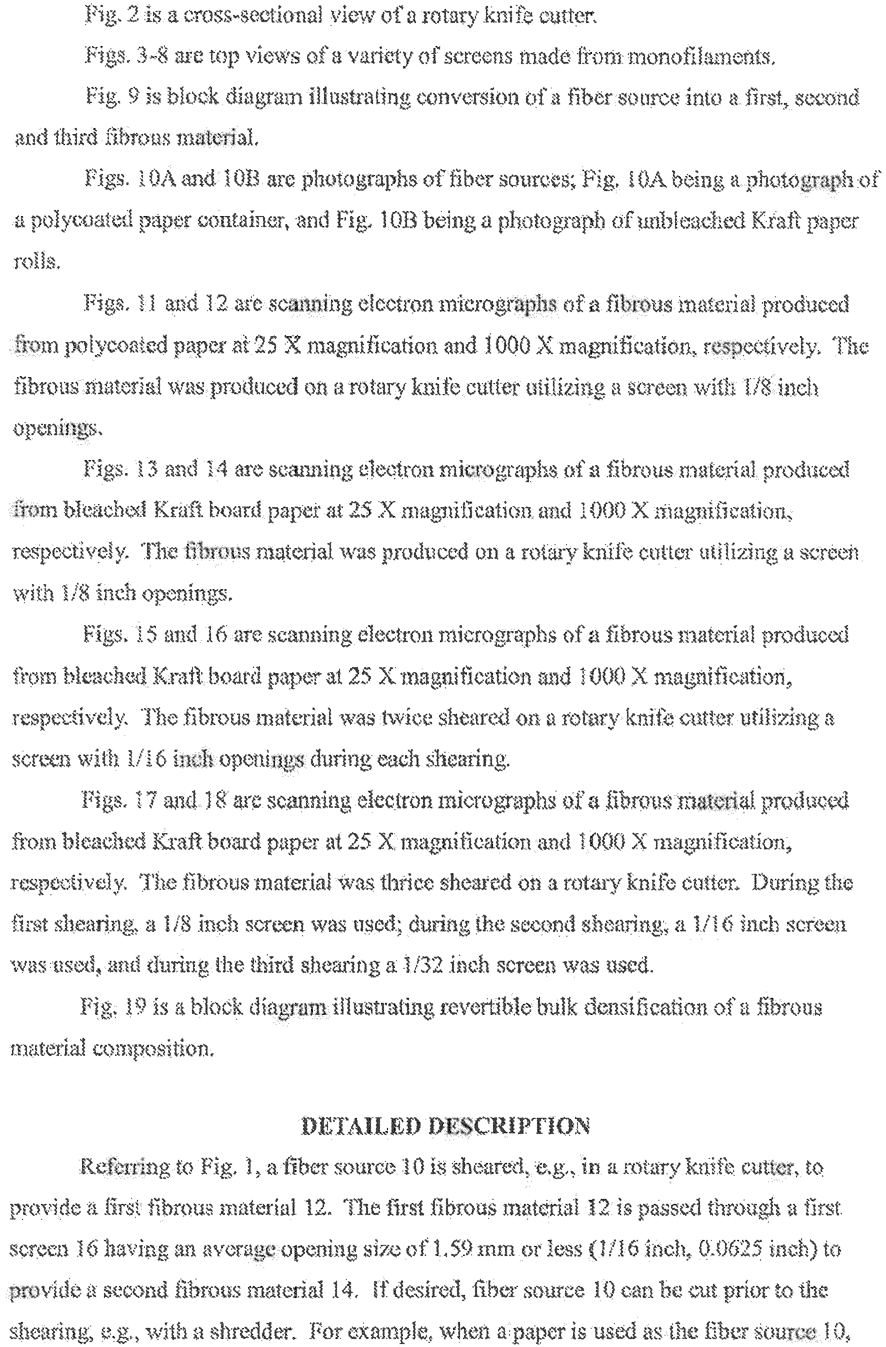

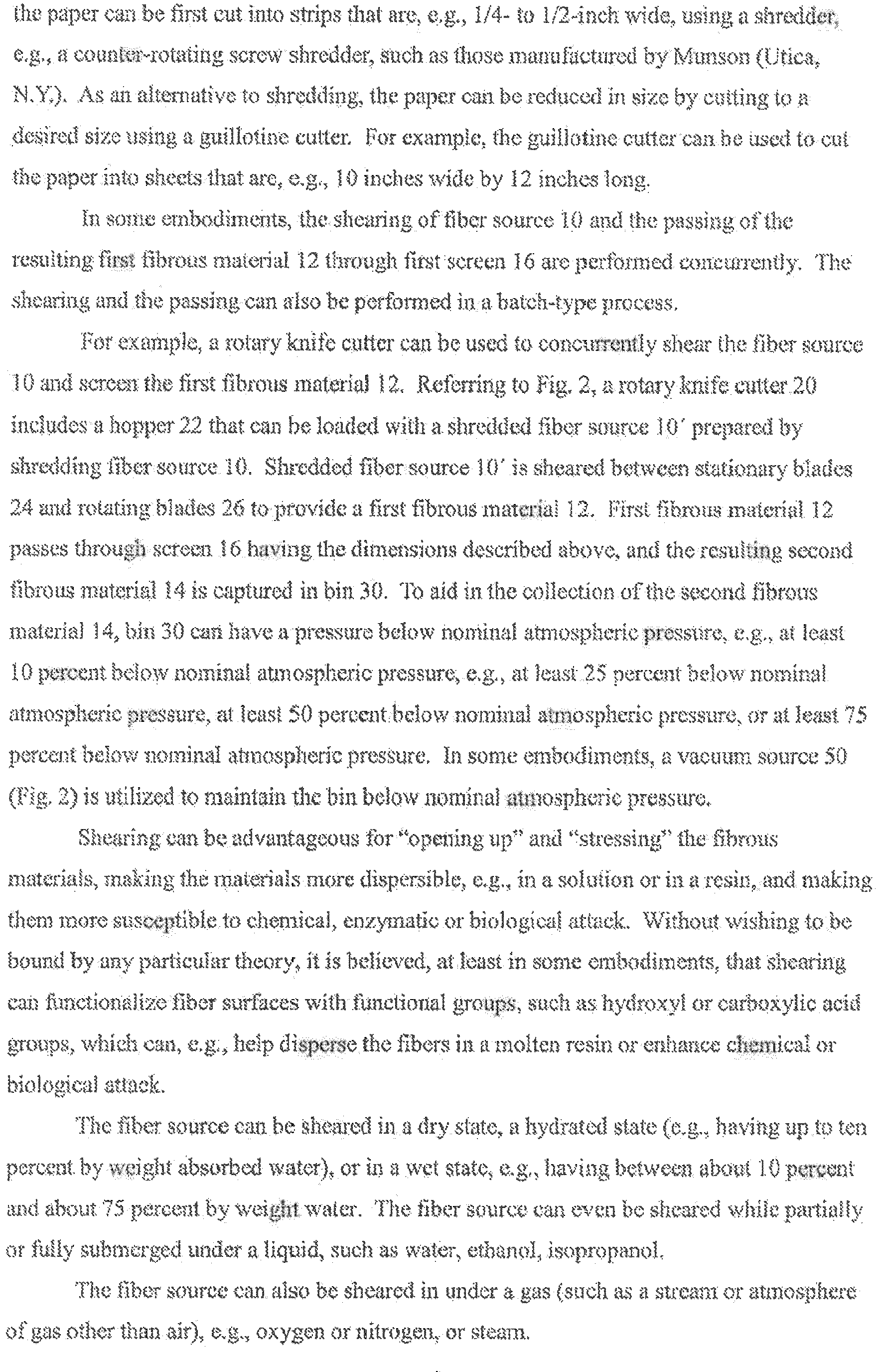

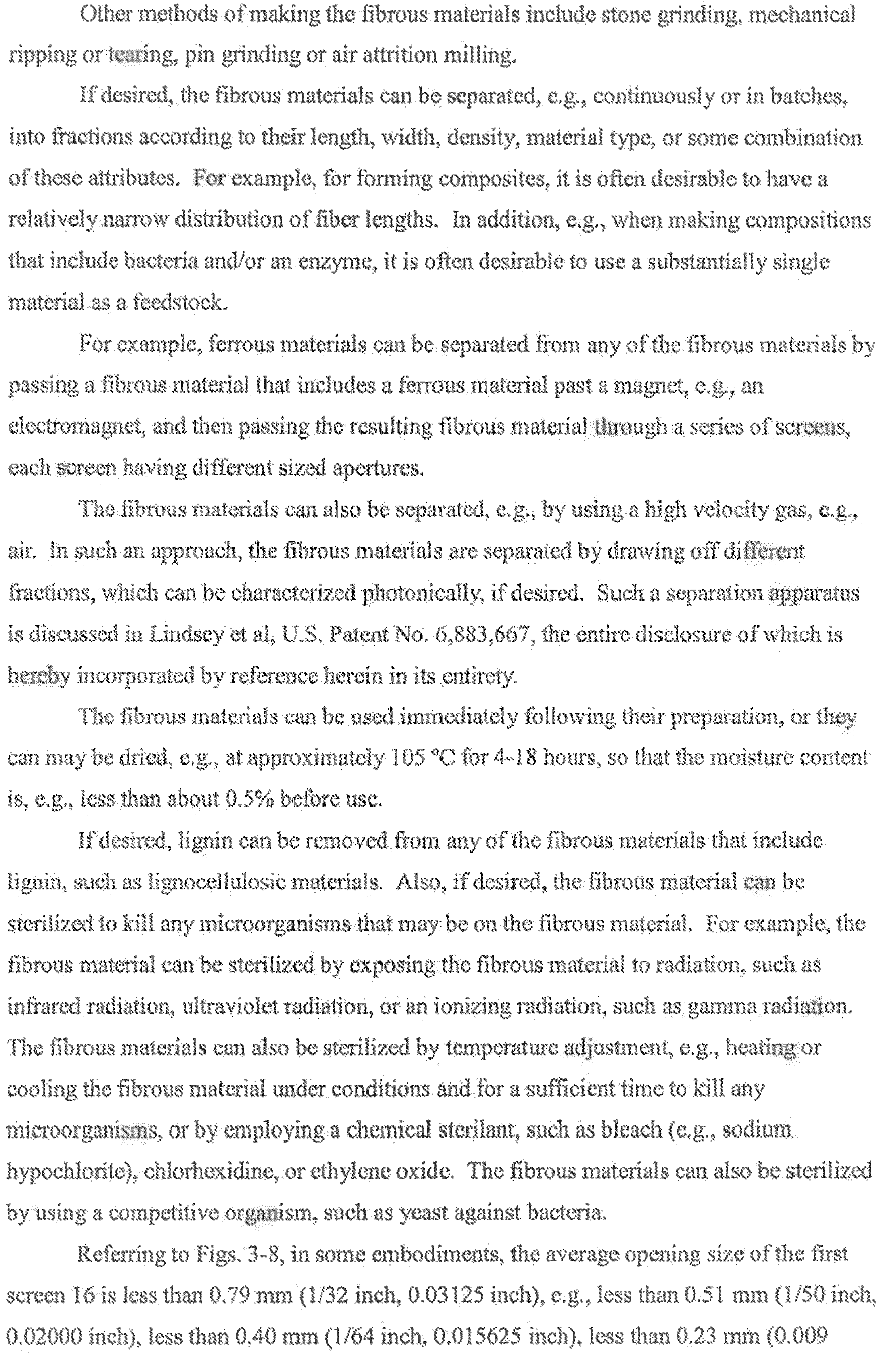

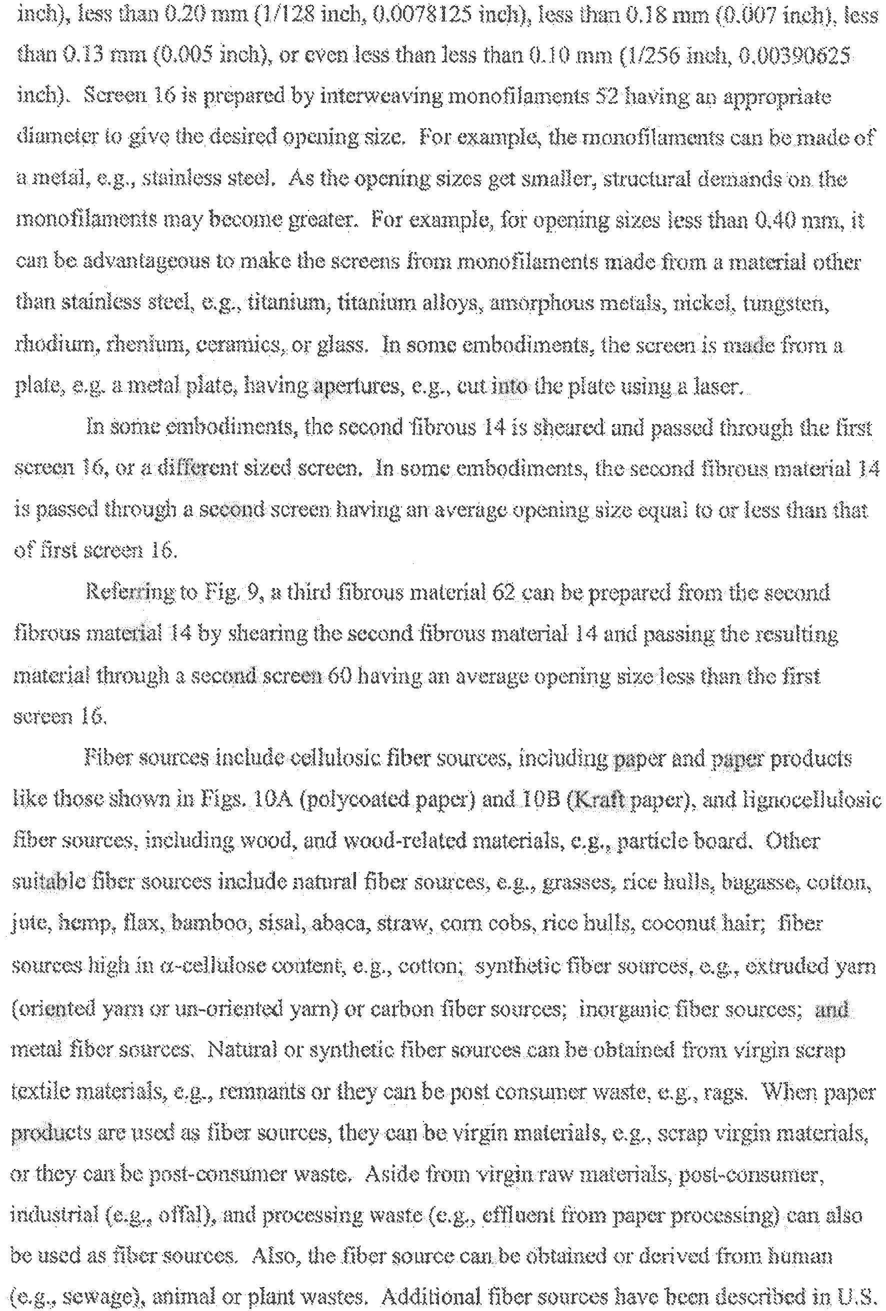

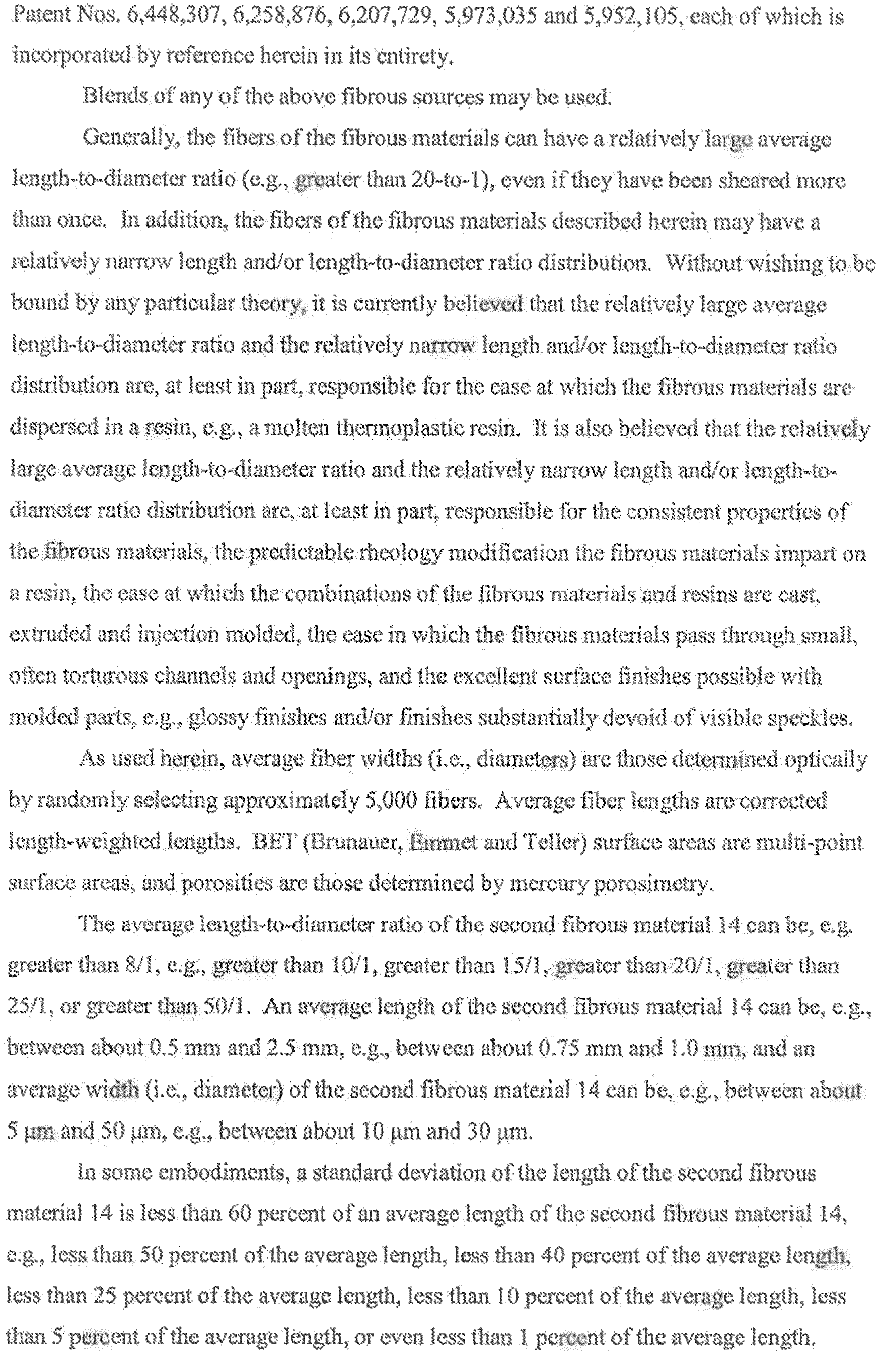

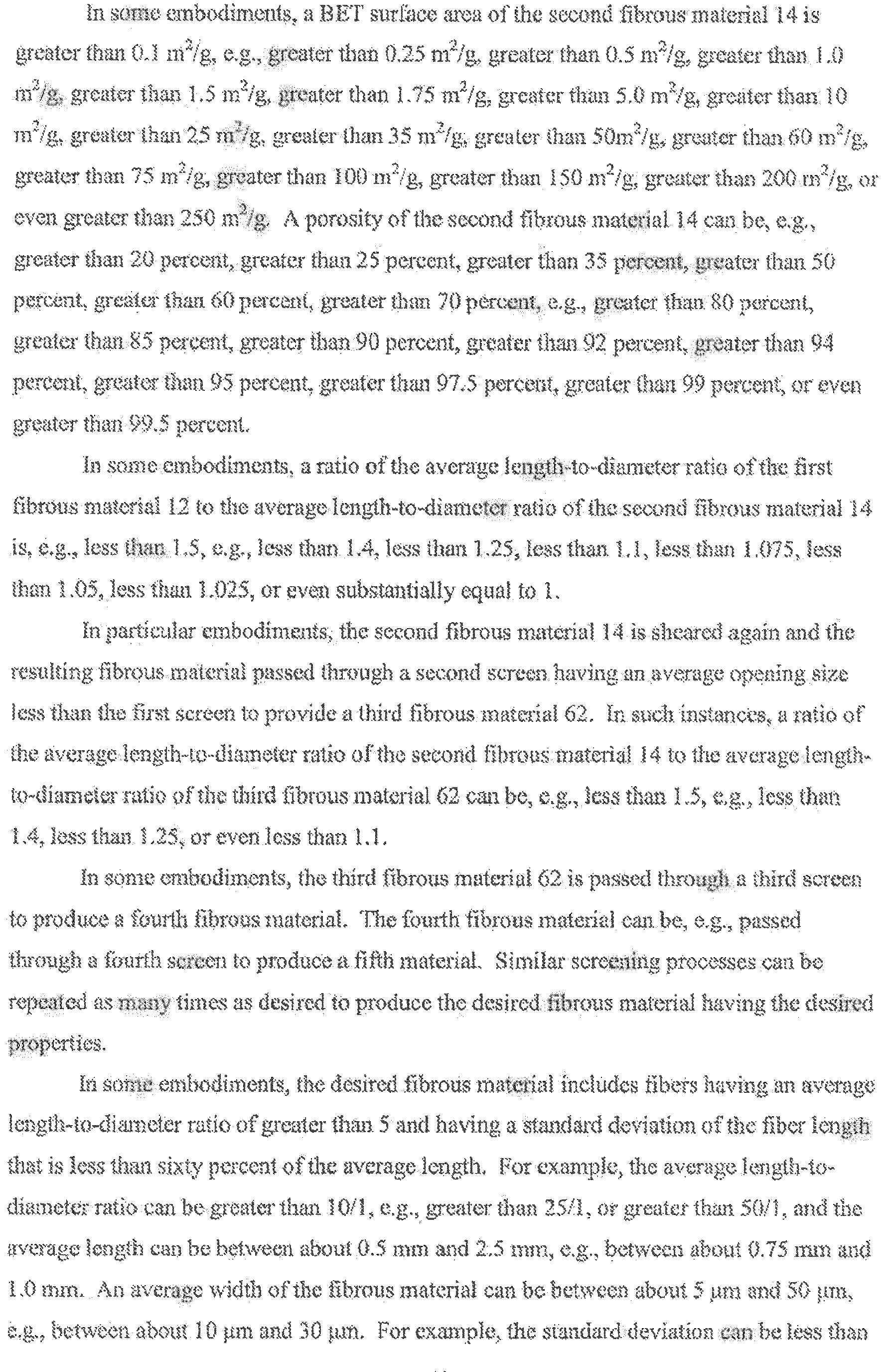

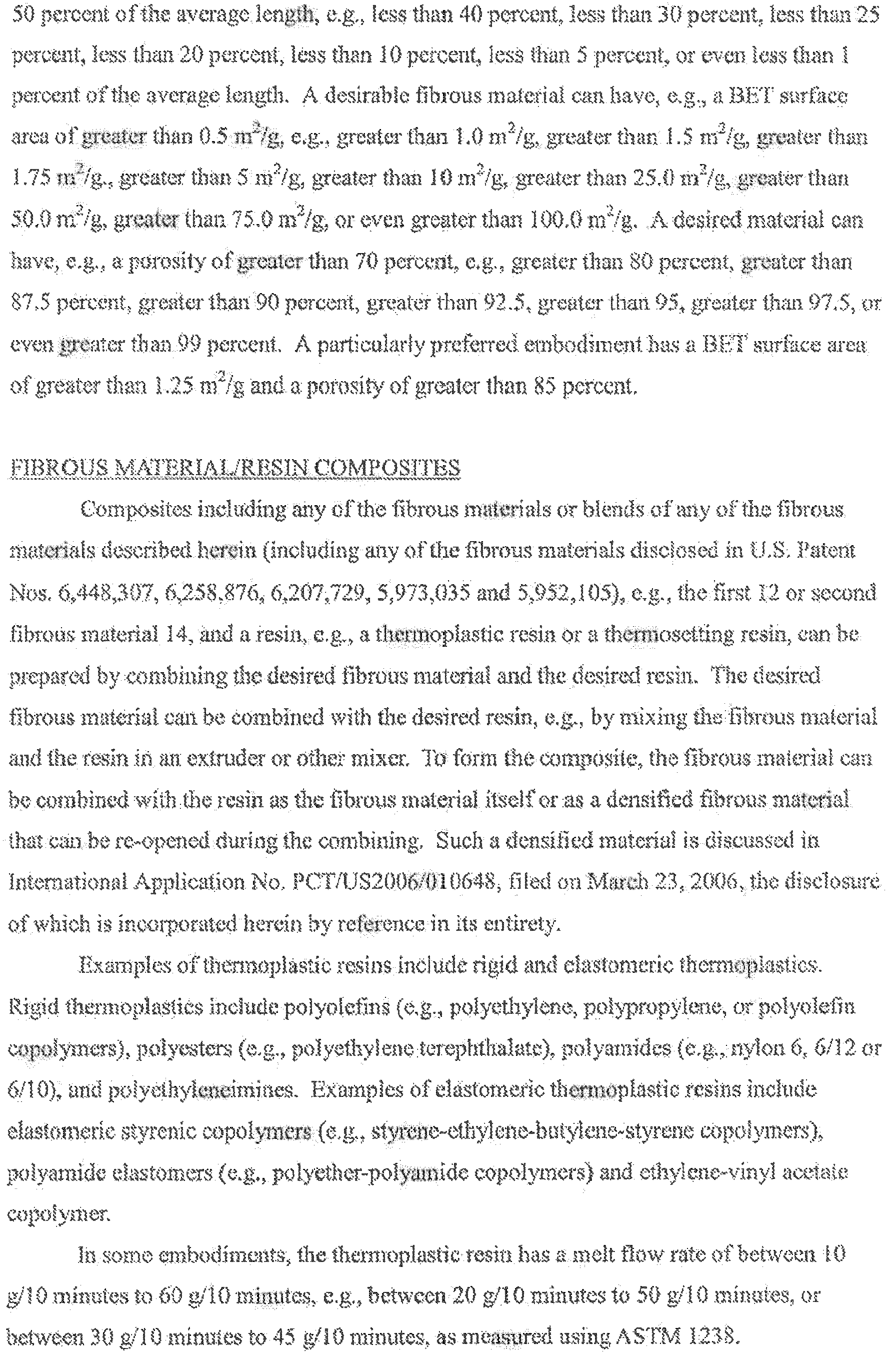

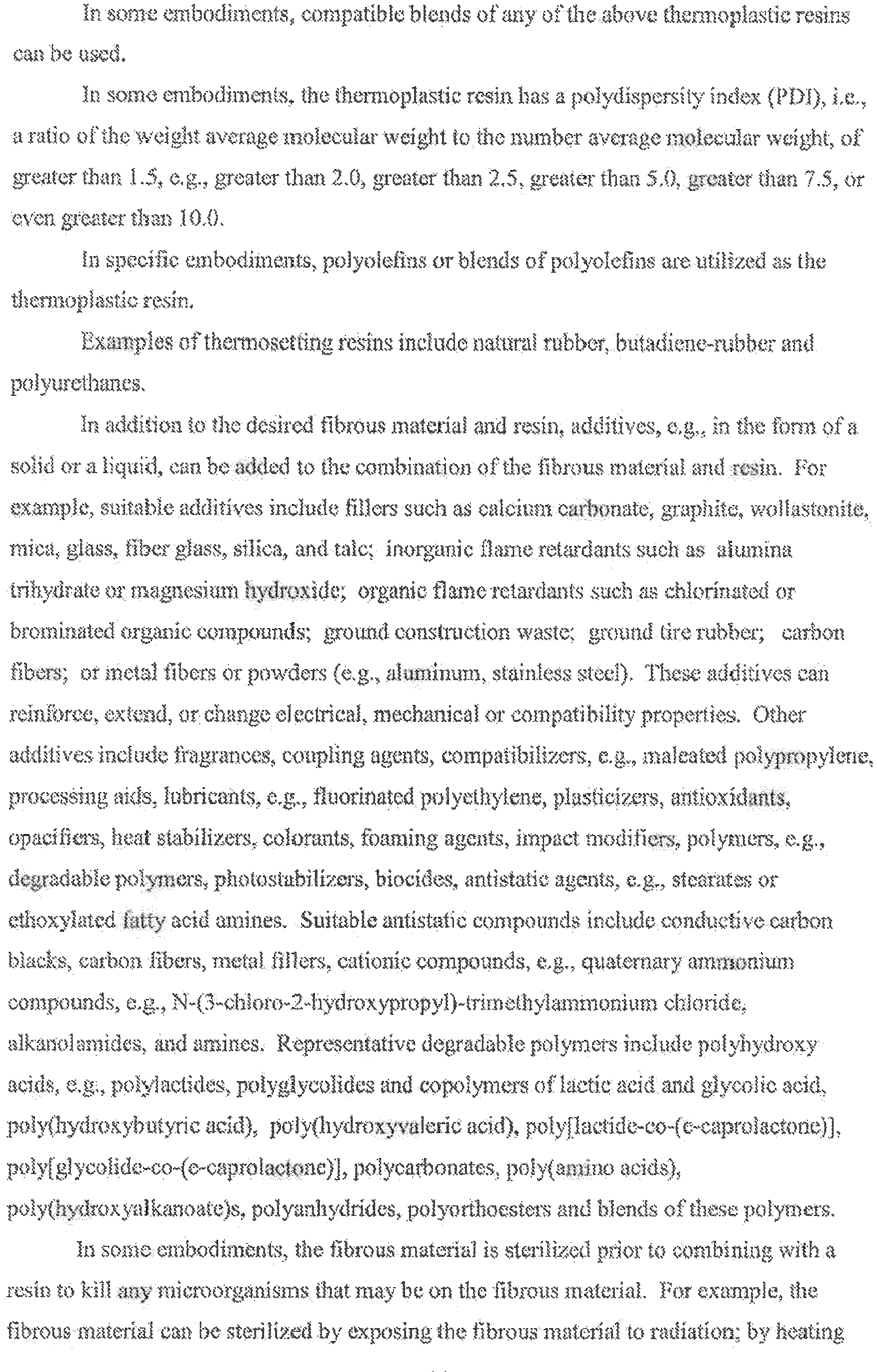

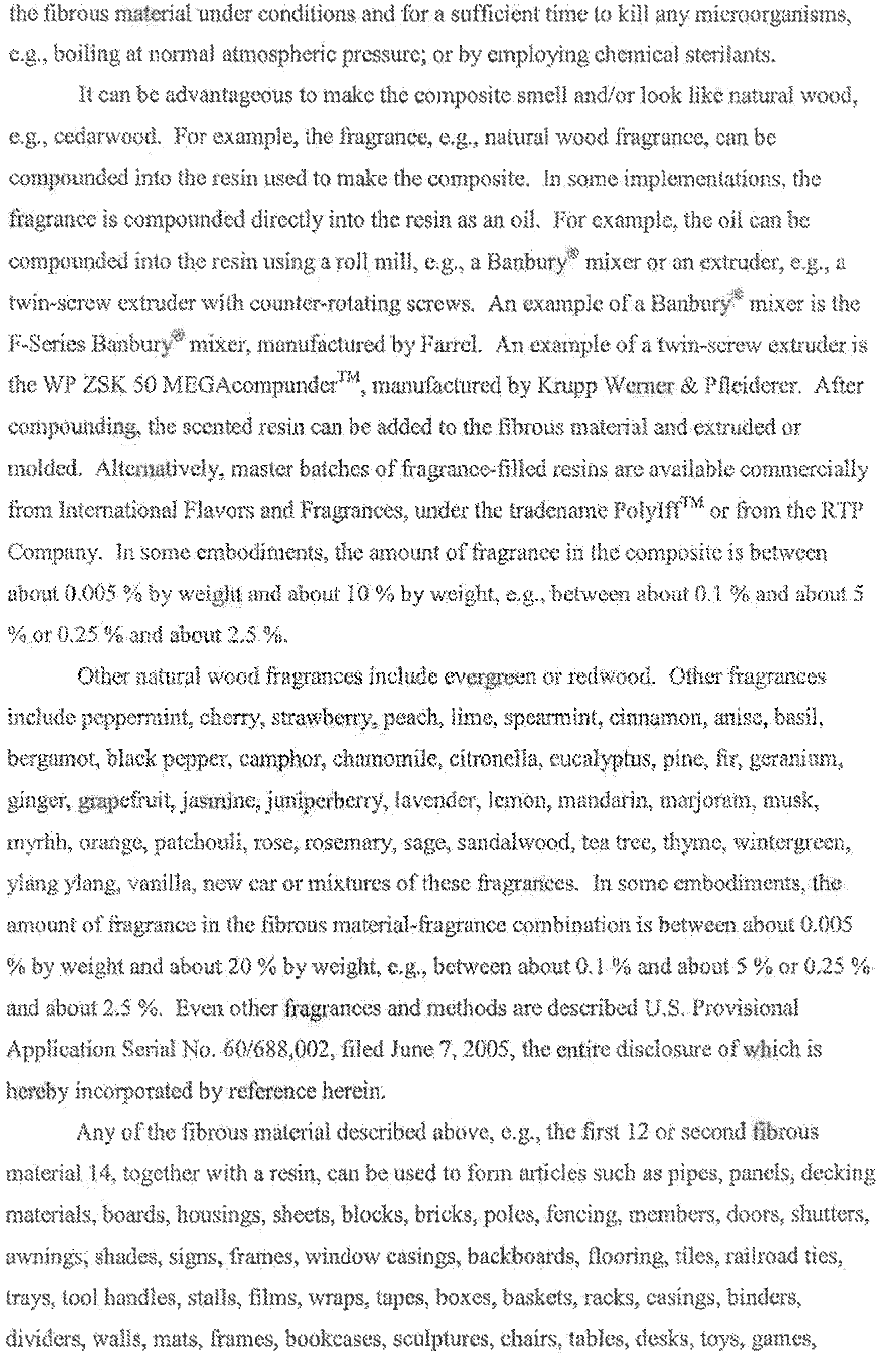

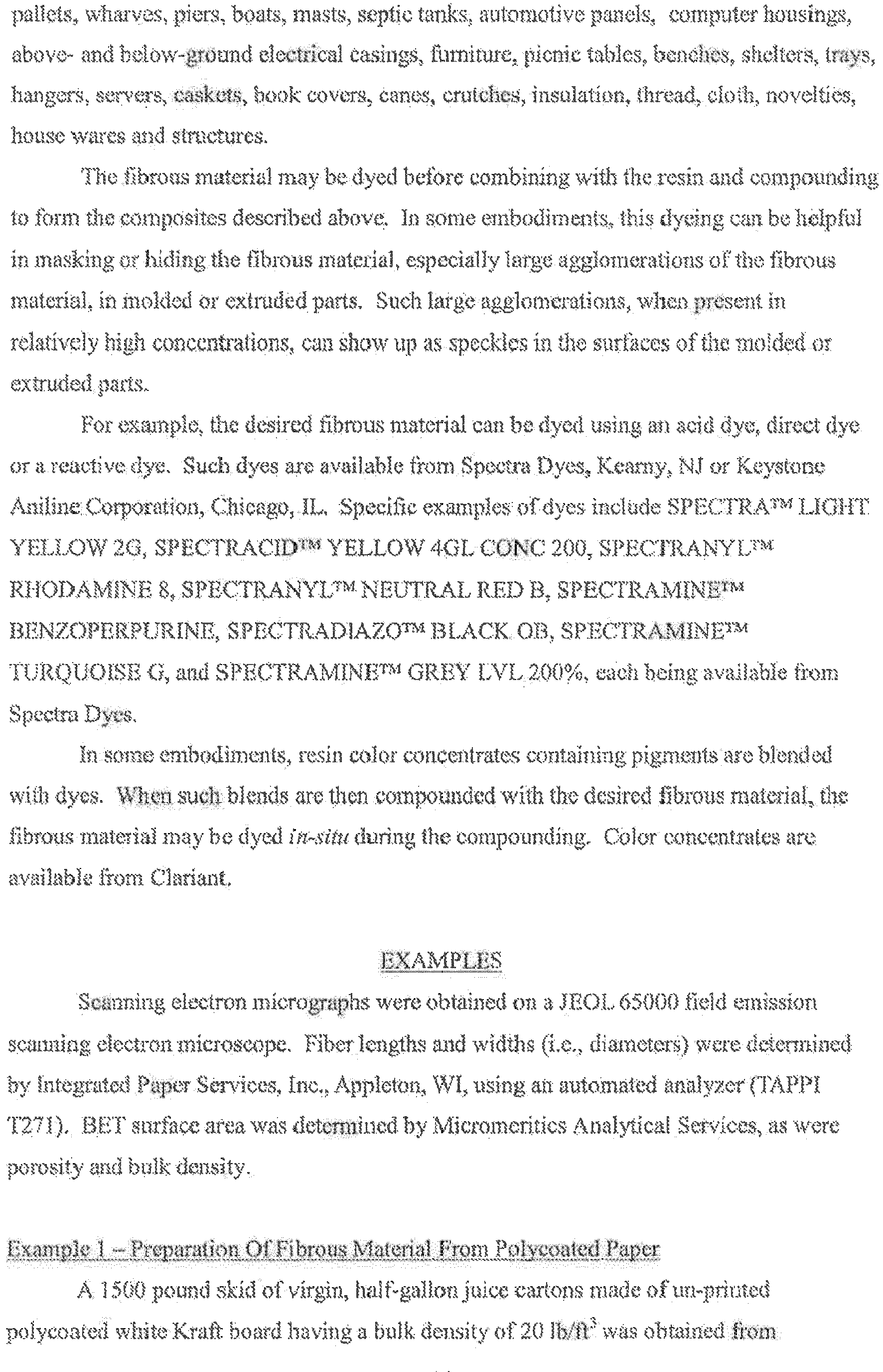

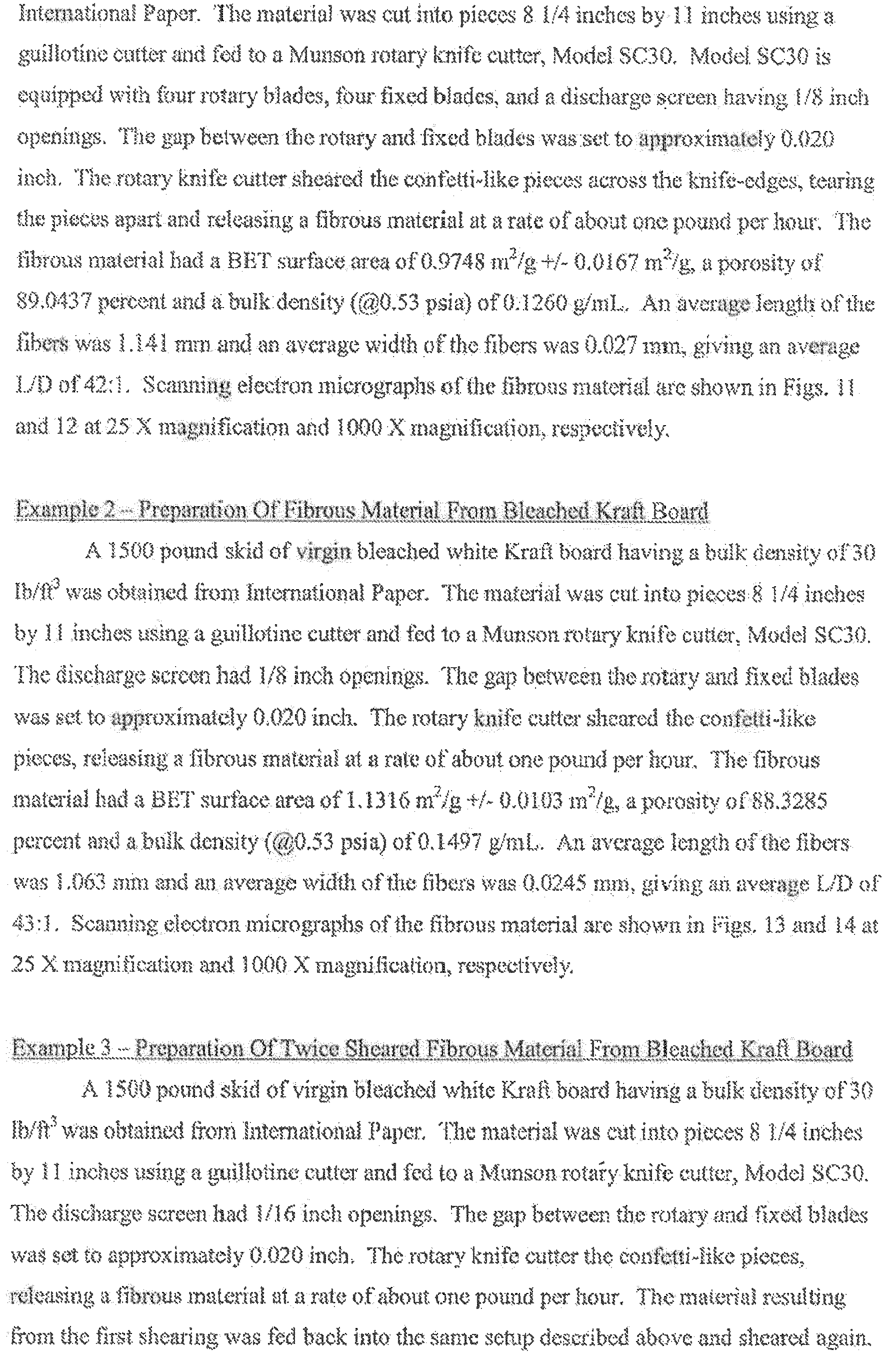

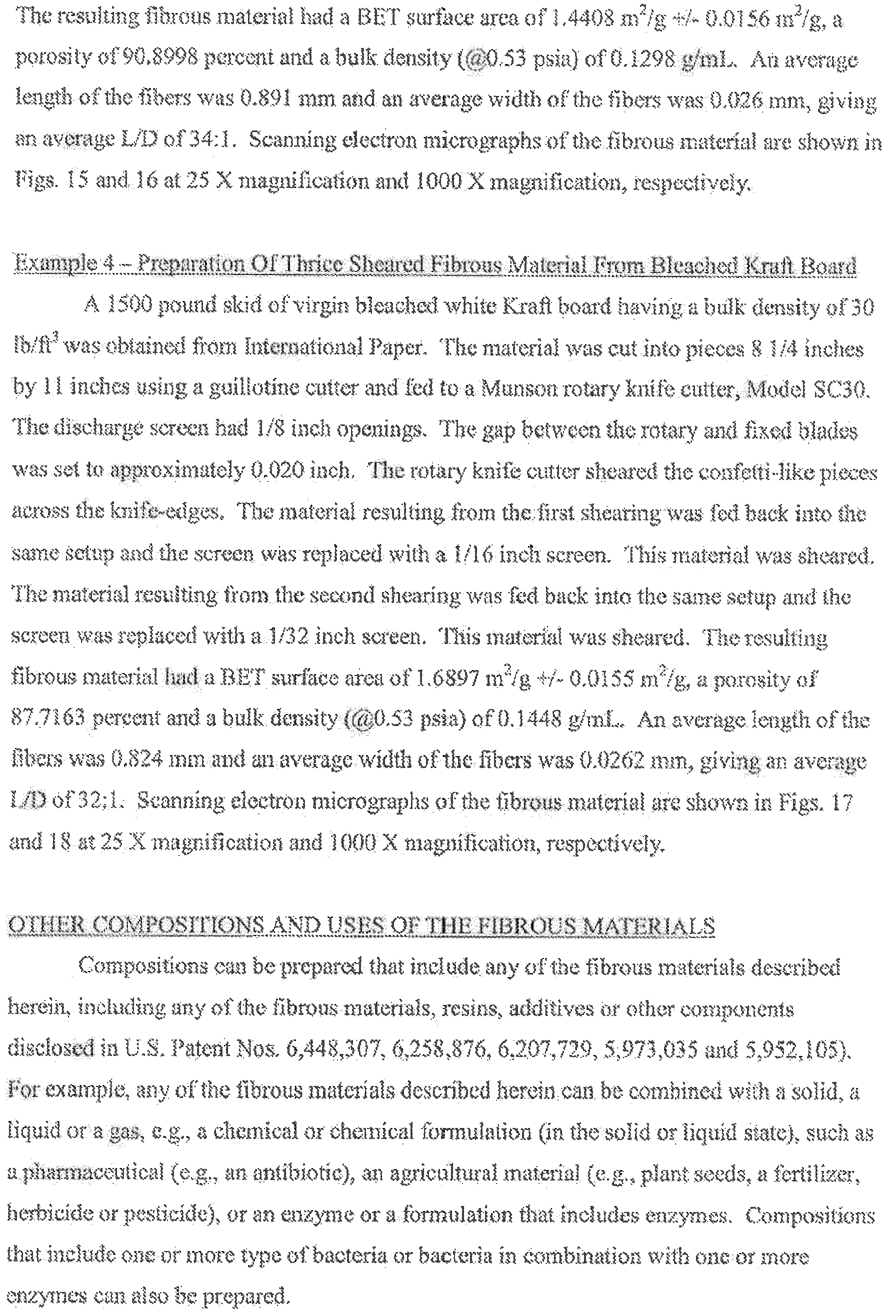

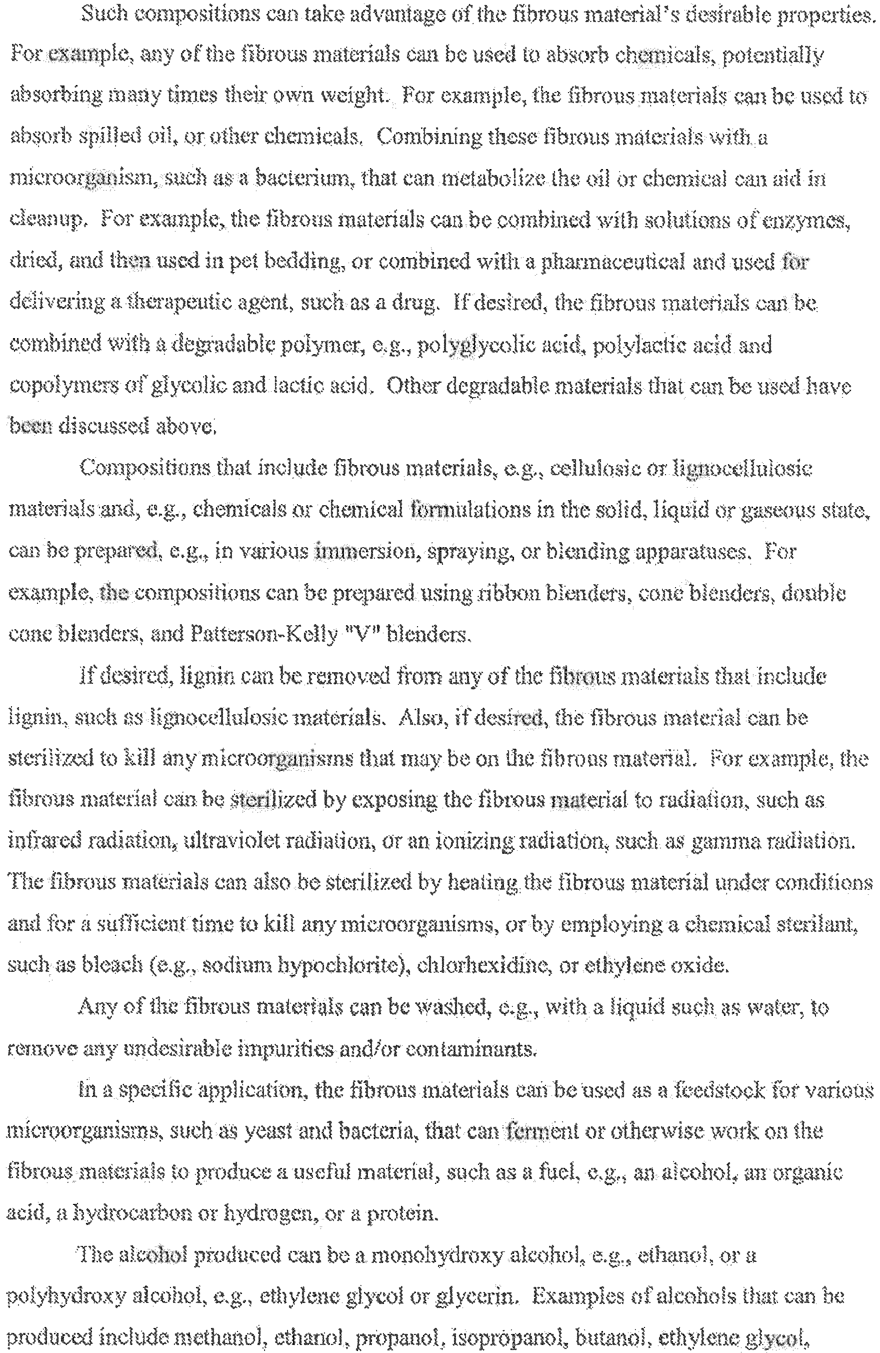

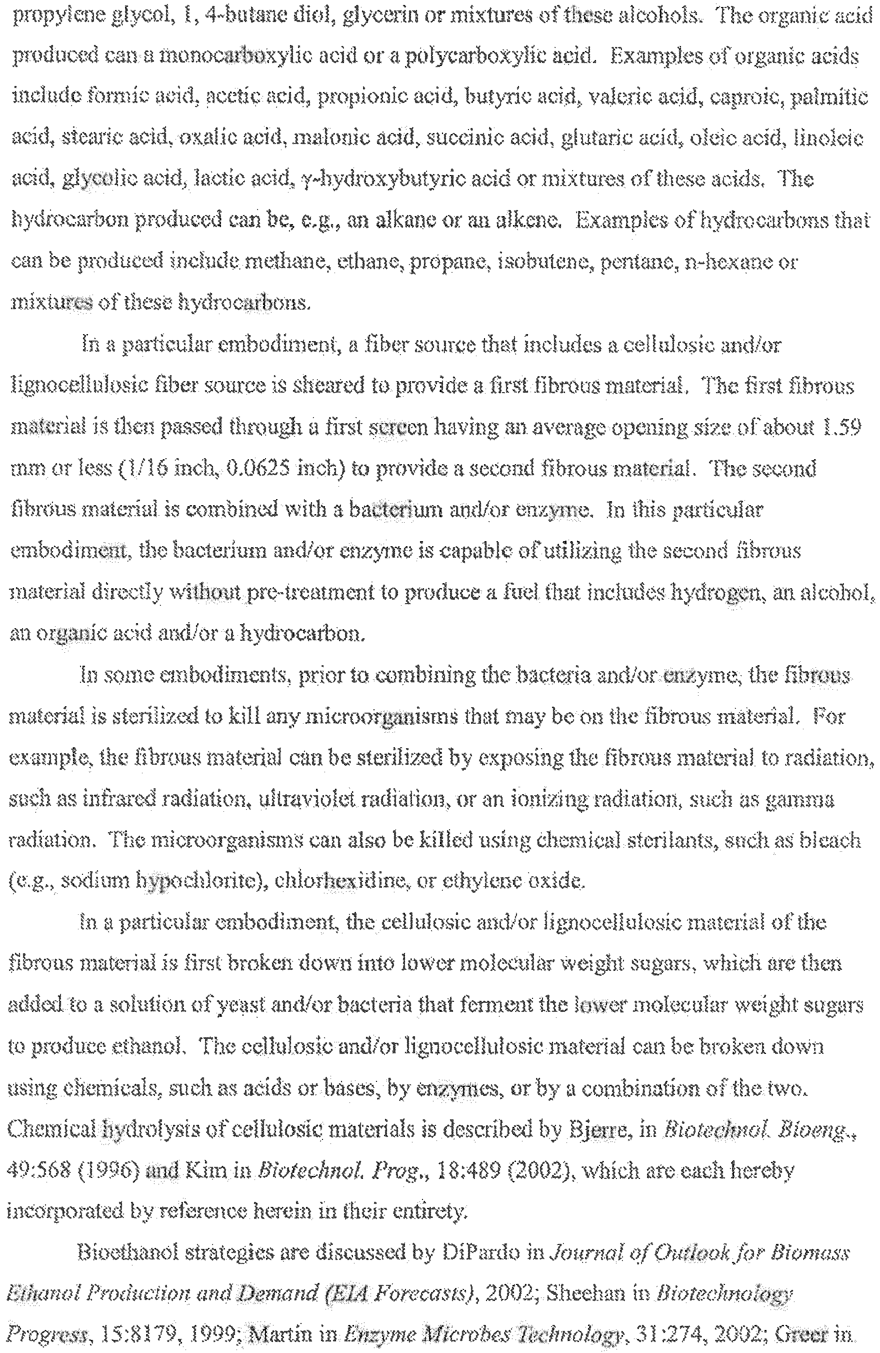

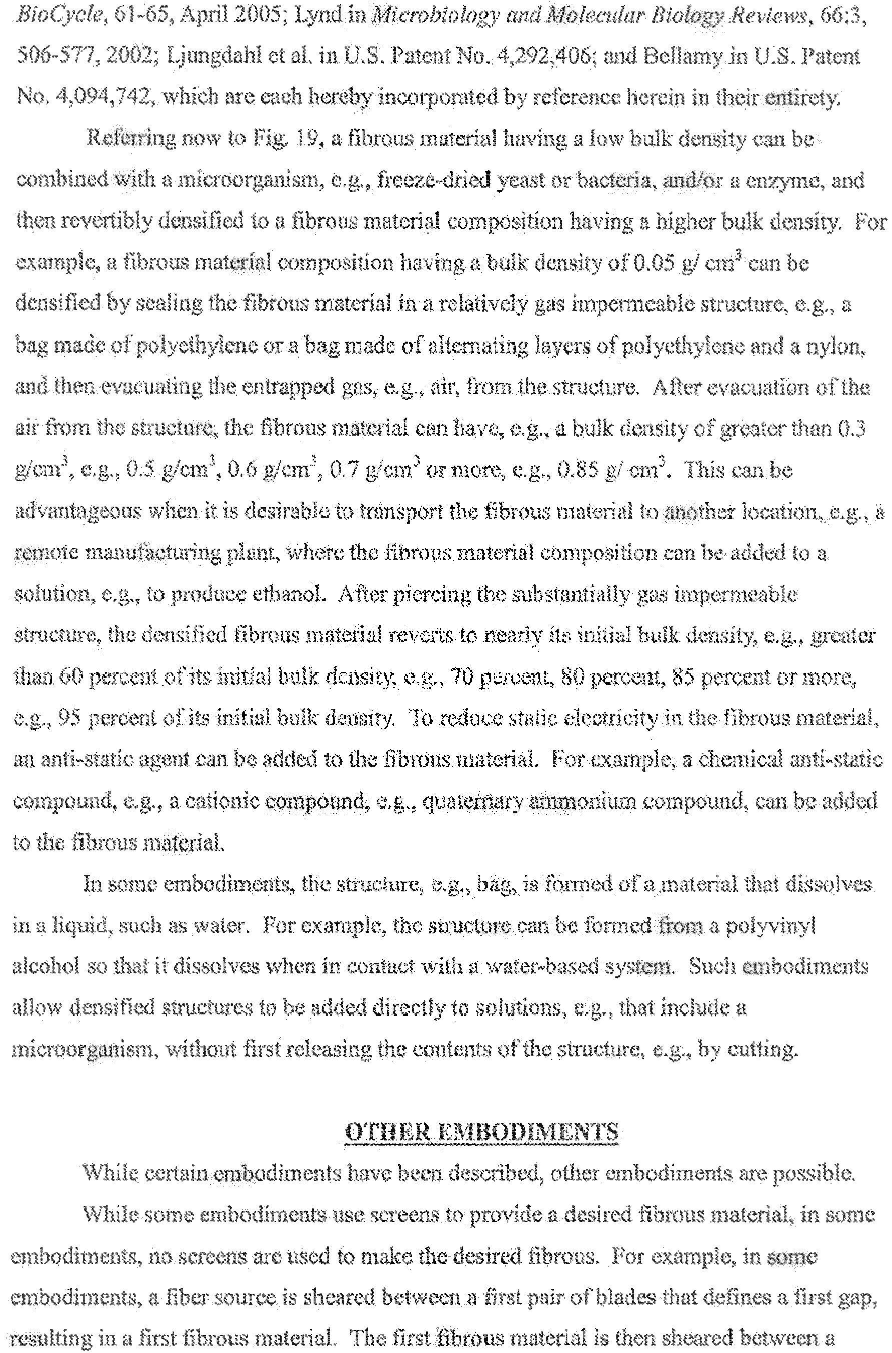

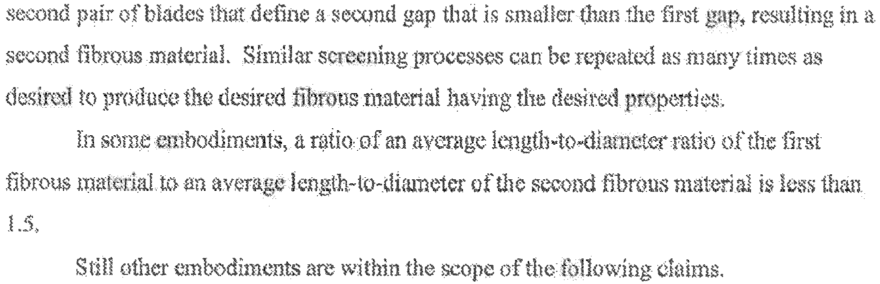

The following numbered paragraphs further describe aspects and embodiments of the invention:
1. A method of making a fuel, the method comprising:
   shearing a fiber source to provide a first fibrous material;
   passing the first fibrous material through a first screen having an average opening size of about 1.59 mm or less (1/16 inch, 0.0625 inch) to provide a second fibrous material; and
   combining the second fibrous material with a bacterium and/or enzyme, the bacterium and/or enzyme utilizing the second fibrous material to produce a fuel comprising hydrogen, an alcohol, an organic acid and/or a hydrocarbon.
2. The method of paragraph 1, wherein the alcohol is selected from the group consisting of methanol, ethanol, propanol, isopropanol, butanol, ethylene glycol, propylene glycol 1,4-butane diol, glycerin, and mixtures thereof.
3. The method of paragraph 1, wherein the organic acid is selected from the group consisting of formic acid, acetic acid, propionic acid, butyric acid, valeric acid, caproic, palmitic acid, stearic acid, oxalic acid, malonic acid, succinic acid, glutaric acid, oleic acid, linoleic acid, glycolic acid, lactic acid, γ-hydroxybutyric acid and mixtures thereof.
4. The method of paragraph 1, wherein the hydrocarbon is selected from the group consisting of methane, ethane, propane, isobutene, pentane, n-hexane, and mixtures thereof.
5. The method of paragraph 1, wherein the second fibrous material has a BET surface area of greater than about 0.25 m²/g.
6. The method of paragraph 1, wherein the second fibrous material has a BET surface area of greater than about 1.25 m²/g.
7. The method of paragraph 1, wherein the second fibrous material has a porosity of greater than about 85 percent.
8. A method of making a fuel, the method comprising;
   shearing a fiber source to provide a first fibrous material; and
   passing the first fibrous material through a first screen having an average opening size of about 1.59 mm or less (1/16 inch. 0.0625 inch) to provide a second fibrous material;
   hydrolyzing the second fibrous material to provide a hydrolyzed material; and
   combining the hydrolyzed material with bacterium and/or enzyme, the bacterium and/or enzyme utilizing the hydrolyzed material to produce a fuel comprising hydrogen, an alcohol, an organic acid and/or a hydrocarbon.
9. A method of densifying a fibrous composition, the method comprising:
   shearing a fiber source to provide a fibrous material;
   combining the fibrous material with a bacterium and/or enzyme to provide a fibrous material composition;
   encapsulating the composition in a substantially gas impermeable material; and
   removing entrapped gas from the encapsulated composition to densify the composition.
10. The method of paragraph 9, wherein the substantially gas impermeable material is soluble in water.
11. The method of paragraph 10, wherein the substantially gas impermeable material is in the form of a bag.
12. The method of paragraph 9, wherein after removing entrapped gas, the fibrous material has a bulk density of greater than about 0.6 g/cm³.

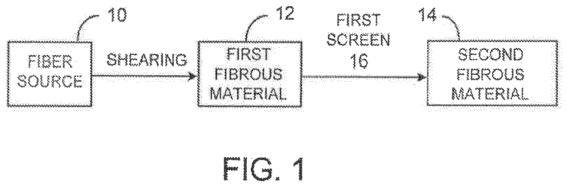

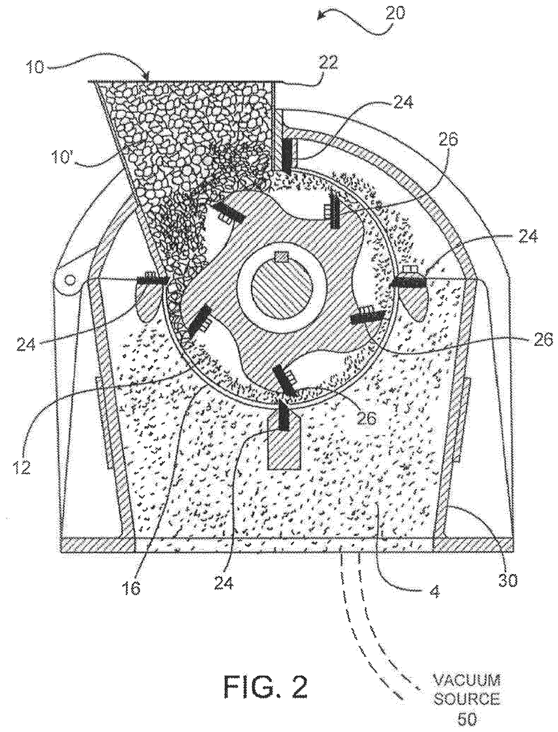

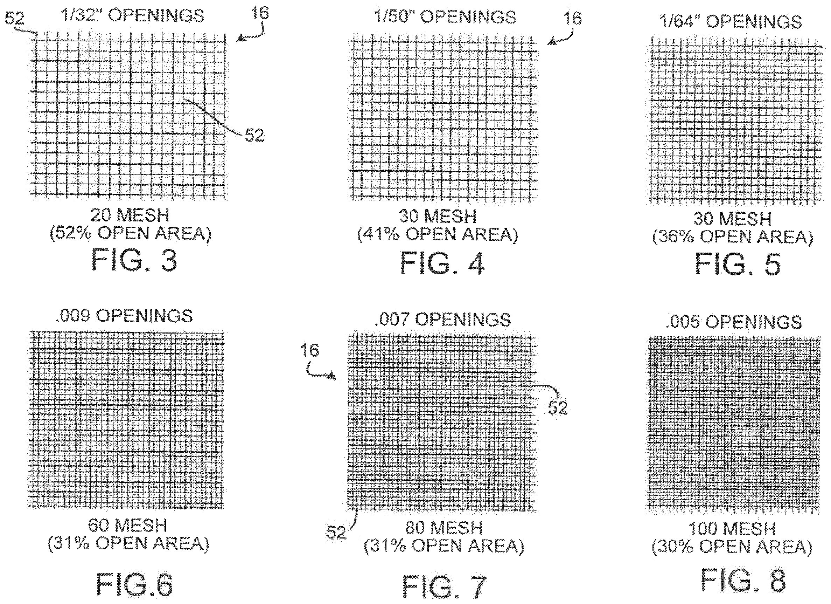

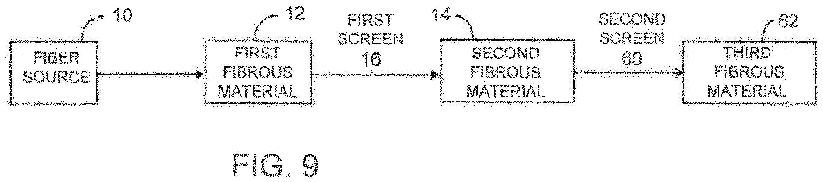

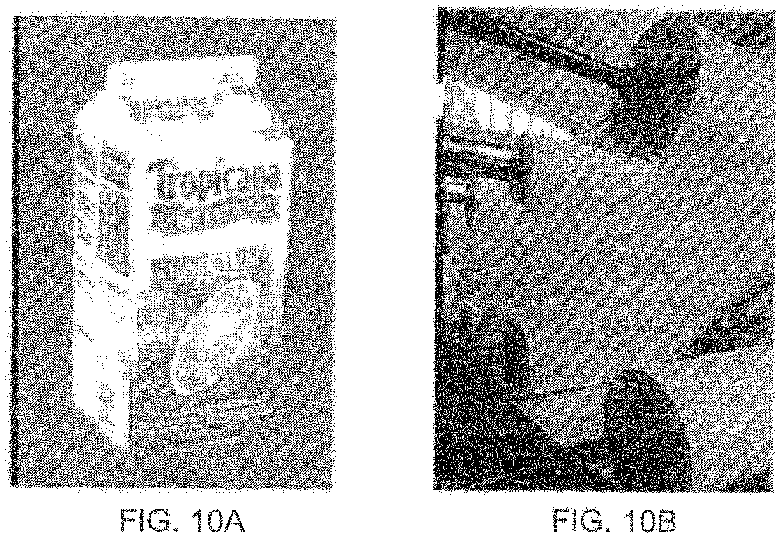

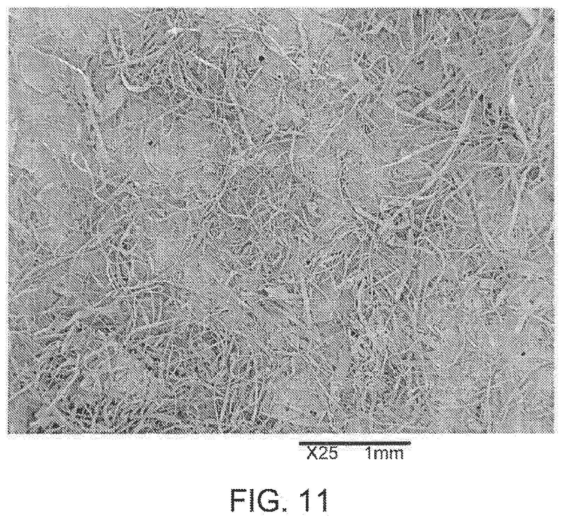

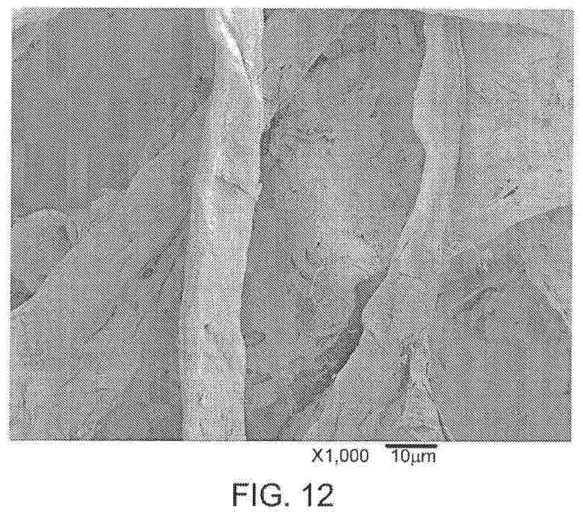

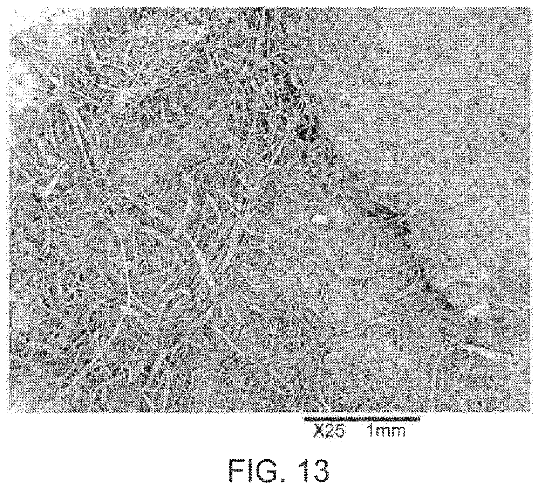

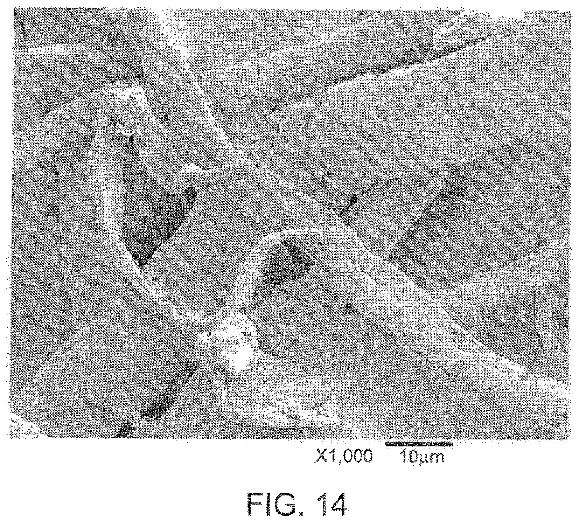

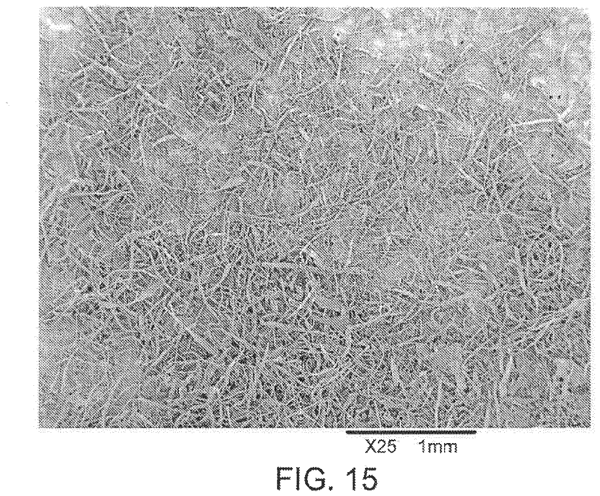

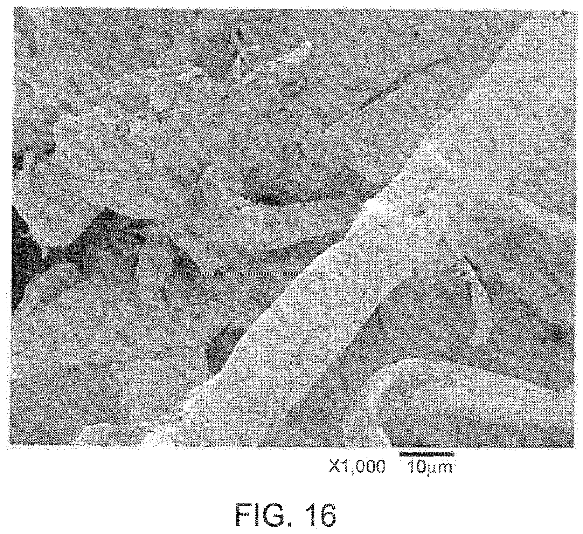

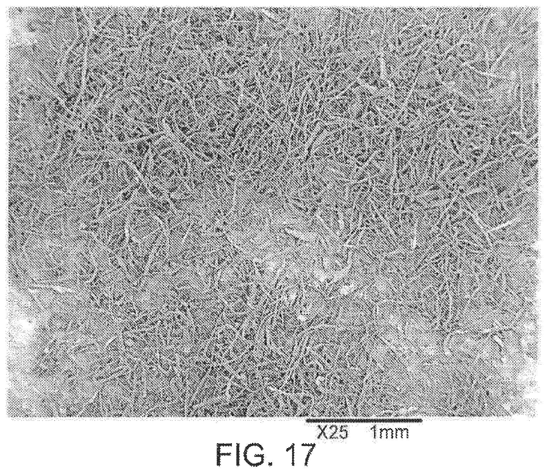

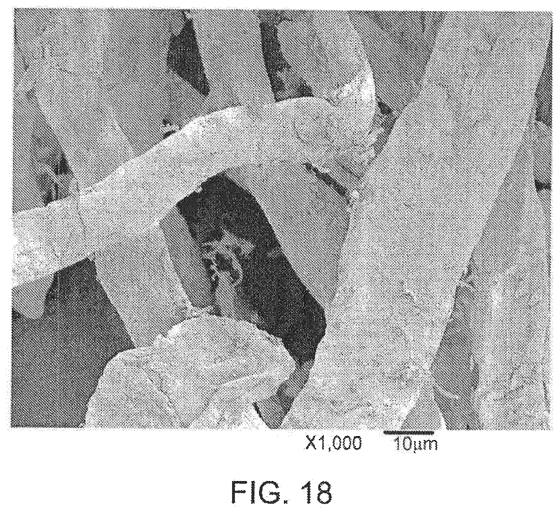

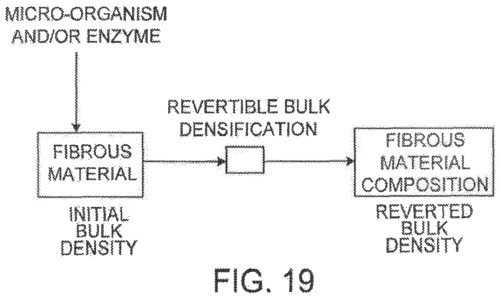

## Claims

1. A method comprising:
mechanically treating a lignocellulosic or cellulosic material and passing the mechanically treated material through a first screen having an opening size of 3.175 mm (1/8 inch) or less, to provide a screened fibrous material, and
combining the screened fibrous material with a biodegradable polymer.

2. The method of claim 1, wherein the degradable polymer is selected from the group consisting of polylactides, polyglycolides and copolymers of lactic acid and glycolic acid, poly(hydroxybutyric acid), poly(hydroxyvaleric acid), poly[lactide-co-(epsilon-capro-55 lactone)], poly[glycolide-co-(epsilon-caprolactone)], polycarbonates, poly(amino acids), poly(hydroxyalkanoate)s, polyanhydrides, polyorthoesters and blends of these polymers.

3. The method of claim 1 further comprising combining the screened fibrous material with a resin.

4. The method of claim 3, wherein the resin is a thermoplastic or thermosetting resin.

5. The method of claim 4, wherein the thermosetting resin is selected from the group comprising natural rubber, butadiene-rubber and polyurethanes.

6. The method of claim 4, wherein thermoplastic resin is a rigid thermoplastic.

7. The method of claim 6, wherein the rigid thermoplastic is selected from the group consisting of polyolefins, polyesters, polyamides and polyethyleneimines.

8. The method of claim 4, wherein the thermoplastic is an elastomeric thermoplastic.

9. The method of claim 8, wherein the elastomeric thermoplastic resins include elastomeric styrenic copolymers, polyamide elastomers, polyether-polyamide copolymers and ethylenevinyl acetate copolymer.

10. The method of claim 1, wherein the screened fibrous material and degradable polymer are combined using an extruder.

11. The method of claim 1 further forming the combined material into a product selected from the group consisting of a stepping stool, pipes, panels, decking materials, boards, housings, sheets, blocks, bricks, poles, fencing, members, doors, shutters, awnings, shades, signs, frames, window casings, back boards, flooring, tiles, railroad ties, trays, tool handles, stalls, films, wraps, tapes, boxes, baskets, racks, casings, binders, dividers, walls, mats, frames, bookcases, sculptures, chairs, tables, desks, toys, games, pallets, wharves, piers, boats, masts, septic tanks, automotive panels, computer housings, electrical casings, furniture, picnic tables, benches, shelters, trays, hangers, servers, caskets, book covers, canes and crutches.

12. The method of claim 1, wherein forming the product includes one or more of extruding, molding, heating, cooling or compressing the combined material.

13. The method of claim 1, wherein the cellulosic or lignocellulosic material is selected from the group consisting of wood, grasses, rice hulls, bagasse, cotton, jute, hemp, flax, bamboo, sisal, abaca, straw, corn cobs, coconut hair, and paper.

14. The method of claim 1, wherein the screened fibrous material has an average length to diameter ratio of greater than 5 and a standard deviation of the fiber length that is less than sixty percent of the average length.

15. The method of claim 1, wherein the screened fibrous material has a BET surface area of at least 0.5 m²/g.

16. The method of claim 1, wherein mechanically treating comprises shearing.

17. The method of claim 1, wherein mechanically treating comprises grinding or milling.

18. The method of claim 1 further comprising combining the screened fibrous material with a pigment or dye.

19. The method of claim 1 further comprising combing the screened fibrous material with a fragrance.

20. The method of claim 1, wherein the lignocellulosic or cellulosic material is passed through a second screen to form the screened fibrous material.

21. A method of making a composite, the method comprising:
mechanically treating a first fiber source selected from the group consisting of wood, grasses, rice hulls, bagasse, cotton, jute, hemp, flax, bamboo, sisal, abaca, straw, corn cobs, coconut hair, and paper, to produce a fibrous material,
passing the fibrous material through a first screen having an opening size of 3.175 mm (1/8 inch) or less, to provide a screened fibrous material, and
combining the screened fibrous material with a resin and a biodegradable polymer.

22. The method of claim 21, wherein combining includes extruding or mixing.

23. The method of claim 21, wherein the first fiber source is a cellulosic or lignocellulosic material.

24. The method of claim 21, wherein the degradable polymer is selected from the group consisting of polylactides, polyglycolides and copolymers of lactic acid and glycolic acid, poly(hydroxybutyric acid), poly(hydroxyvaleric acid), poly[lactide-co-(epsilon-capro-55 lactone)], poly[glycolide-co-(epsilon-caprolactone)], polycarbonates, poly(amino acids), poly(hydroxyalkanoate)s, polyanhydrides, polyorthoesters and blends of these polymers.

25. The method of claim 21, wherein the resin is a cross-linkable resin.

26. The method of claim 21 further comprising passing the fiber source through a second screen to provide the screened material.

27. The method of claim 21 further comprising extruding, molding, heating, cooling or compressing the combined material.
